**Europäisches Patentamt**

**European Patent Office**

**Office européen des brevets**

(19)

(11) Veröffentlichungsnummer : **0 330 825 B1**

(12)

# EUROPÄISCHE PATENTSCHRIFT

(45) Veröffentlichungstag der Patentschrift :
25.03.92 Patentblatt 92/13

(51) Int. Cl.$^5$ : **A61F 13/02**

(21) Anmeldenummer : **89101014.2**

(22) Anmeldetag : **20.01.89**

(54) Primärverpackung für flächenstabilisierte Verbandstoffe.

(30) Priorität : **29.02.88 DE 3806444**

(43) Veröffentlichungstag der Anmeldung :
**06.09.89 Patentblatt 89/36**

(45) Bekanntmachung des Hinweises auf die
Patenterteilung :
**25.03.92 Patentblatt 92/13**

(84) Benannte Vertragsstaaten :
**AT BE CH DE ES FR GB GR IT LI LU NL SE**

(56) Entgegenhaltungen :
**EP-A- 0 144 891**
**EP-A- 0 171 800**
**DE-A- 1 935 916**
**US-A- 3 277 891**
**US-A- 4 450 844**

(73) Patentinhaber : **LTS Lohmann**
**Therapie-Systeme GmbH & Co. KG**
**Irlicherstrasse 55**
**W-5450 Neuwied 12 (DE)**

(72) Erfinder : **Barth, Peter, Dr.rer.nat.**
**Johann-Gottfried-Herder-Strasse 6**
**W-5450 Neuwied 12 (DE)**
Erfinder : **Hoffmann, Hans-Rainer, Dr.**
**Burghofstrasse 123**
**W-5450 Neuwied 22 (DE)**
Erfinder : **Müller, Walter, Dr.**
**Engerser Strasse 56**
**W-5450 Neuwied 1 (DE)**
Erfinder : **Kindel, Heinrich**
**Westerwaldstrasse 7**
**W-5455 Rengsdorf (DE)**

(74) Vertreter : **Neidl-Stippler, Cornelia, Dr.**
**Rauchstrasse 2**
**W-8000 München 80 (DE)**

## Beschreibung

Die Erfindung betrifft eine Primärverpackung für flächenstabilisierte Verbandstoffe, die eine Trägerschicht und eine Haftklebebereiche aufweisende hautseitige Kontaktfläche, die vor Gebrauch des Verbandstoffes durch eine abziehbare Schutzschicht geschützt ist, und eine an der der Haut abgewandten Oberfläche der Trägerschicht ablösbar angebrachte, mindestens einschichtige Stützfolie enthalten, die vom applizierten Verbandstoff entfernbar ist. Dabei findet die Entfernung der Stützfolie nach Erfüllung ihrer Funktion der Flächenstabilisierung der Trägerschicht vom applizierten Verbandstoff statt.

Die Verbandstoffe lassen sich in zwei große Gruppen einteilen. Die eine Gruppe umfaßt solche, die am Ort ihrer Application durch zusätzliche Vorrichtungen oder Maßnahmen fixiert werden, während bei der anderen Gruppe die Fixierung durch haftklebende Bereiche des Verbandstoffes selbst erfolgt. Bei der letzten Gruppe stieß die Forderung nach dünnen und flexiblen Trägerschichten, die eine brauchbare Wasserdampfdurchlässigkeit und gute Anschmiegsamkeit an die Konturen des Applikationsortes gewährleisten, auf Schwierigkeiten bei der Applikation. So kommt es nach Abziehen der Schutzschicht beispielsweise leicht zum Einrollen der Randbezirke, zum Insichverkleben von Haftklebebereichen und zu Faltenbildung, was zum Unbrauchbarwerden des betreffenden Verbandstoffes führt.

Hier konnte durch Anbringen einer sogenannten Stützfolie zur Flächenstabilisierung (DE-A-19 35 916) eine Verbesserung erzielt werden, wobei eine gegenüber der Trägerschicht starrere Schicht mit dieser so verbunden ist, daß sie nach der Applikation abgelöst werden kann. Dieses Prinzip hat sich inzwischen bewährt (vergleiche DE-C- 33 44 334 bzw. EP-A-0 144 891).

Als nachteilig muß allerdings angesehen werden, daß die Stützfolie nach Ablösen vom applizierten Verbandstoff verworfen wird und damit in ihrer Funktion auf die Flächenstabilisierung beschränkt wird. Der Aufwand bei der Materialbereitstellung und den Herstellungsmaßnahmen für dieses zusätzliche Element derartiger Verbandstoffe muß als nicht unerheblich bezeichnet werden.

Es ist daher Aufgabe der Erfindung, die Funktion der Stützfolie so zu erweitern, daß das Aufwand-Nutzen-Verhältnis günstiger wird.

Die Aufgabe der Erfindung wird durch eine gattungsgemäße Primärverpackung gelöst, bei der die Stützfolie oder die Stützfolie und die Schutzschicht jeweils ein Teil einer den Verbandstoff schützenden Verpackung ist.

Aus der US-A-4 450 844 ist ein Epikutan-Testpflaster ohne Stützfolie bekanntzeworden, bei dem in einem Gehäuse aus flexiblem Material ein Wirkstoff-Reservoir auf genommen ist. Dieses Gehäuse wird mittels eines hafs - Klebenden Flächengebildes and der Haus randflähis fixiert. Das bekannte Pfeaster weist eine vor der Applikation abgiehbare Abdeckfolie auf.

Unter Primärverpackung wird im Sinne der Erfindung eine unmittelbar den Verbandstoff umhüllende Schicht verstanden, wobei die derart verpackten Einzelstücke durch Sekundär- und Tertiärverpackung zu größeren Einheiten zusammengefaßt werden können. Der eigentliche Schutz des Substrats wird dabei in erster Linie durch die Primärverpackung gewährleistet. Hierdurch können Verpackungsteile und damit um weltbelastende Abfälle vermieden werden. Vorteilhafte Weiterbildungen ergeben sich auch aus den Unteransprüchen.

Die Erfindung ist bei allen durch Stützfolien flächenstabilisierten Verbandstoffen anwendbar. Diese Verbandstoffe sind bekannt und werden bspw. als Wundverbände, Fixierverbände wirkstoffabgebende Pflaster- und Inzisionsfolien eingesetzt. Die Trägerschicht des Verbandstoffes kann beispielsweise ein textiles Material, ein Polymermaterial oder eine Metall enthaltende Schicht sein. Als Stützfolie wird ein mindestens einschichtiges Flächengebilde aus Polymere, Papier oder Metall enthaltenden Materialien eingesetzt. Sie hat flächenmäßig mindestens dieselbe Ausdehnung wie die Trägerschicht, kann aber bei einer bevorzugten Ausgestaltung der Erfindung an einer oder mehreren Seiten über den Rand der Trägerschicht hinausragen. Die lösbare Verbindung zwischen Stützfolie und Trägerschicht kann durch bekannte Techniken erzeugt werden, wie beispielsweise Erzeugen der Trägerschicht unmittelbar auf der Stützfolie, Heißsiegelung, Verschweißung, Verklebung, Verprägung oder Ausbildung von elektrostatischen Anziehungskräften.

Die Materialien zur Herstellung der den Kontakt mit der Haut sicherstellenden Haftklebebereiche sind aus der Fülle der Möglichkeiten unter dem Gesichtspunkt der physiologischen Unbedenklichkeit ausgewählt. Eine abziehbare Schutzschicht schützt die Hautkontaktfläche des Verbandstoffes vor Gebrauch.

Der Sinn und Zweck einer Primärverpackung eines solchen Verbandstoffes sind dem Fachmann bekannt. Die bisherige Lösung, den Verbandstoff als Ganzes in eine ihn auf allen Seiten umschließende Hülle einzubringen, die dann an allen Rändern durch beispielsweise Verklebung, Versiegelung, Verschweißung oder Verprägung den Erfordernissen entsprechend verschlossen wird, befriedigt wegen des notwendigen Materialeinsatzes nicht.

Gemäß der Erfindung wird eine Fläche der Primärverpackung durch die Stützfolie gebildet, an deren Ober- oder Unterseite im Randbereich das den übrigen Teil der Verpackung bildende Flächengebilde befestigt wird.

Die Befestigung kann beispielsweise durch Heißsiegeln, Verschweißen, Verprägen oder Verkleben erfolgen. Ist die Stützfolie flächenmäßig der Trägerschicht gleich, so ist ein Befestigen der übrigen Verpackungsbestandteile an der Oberseite der Stützfolie vorgesehen. Überragt die Stützfolie die Trägerschicht, so kann es bevorzugt sein, die Befestigung an der Unterseite der überragenden Teile der Stützfolie vorzusehen.

Nach Aufreißen der beutelartigen Primärverpackung verbleibt die Stützfolie an der Rückseite der Trägerschicht, wodurch die erwünschte Stabilisierung des Verbandstoffes bei der Applikation gewährleistet ist.

Die Befestigung des übrigen flächenförmigen Verpackungsmaterials an der Stützfolie und die Herstellung der lösbaren Verbindung zwischen Stützfolie und Trägerschicht können erfindungsgemäß auch in einem Schritt erfolgen.

Die Auswahl des übrigen flächenförmigen Verpackungsmaterials wird durch die jeweiligen Anforderungen des zu verpackenden Verbandstoffes bestimmt, weshalb keine allgemein gültigen Angaben gemacht werden können. In der Regel kommen beispielsweise neben undurchlässigem Papier, Polymere oder Metall enthaltenden Materialien auch solche in Betracht, die von ihrer Struktur her den textilen Flächengebilden zuzuordnen sind. Die Materialauswahl für die Stützfolie und die übrige Verpackung braucht nicht dieselbe zu sein, muß aber auf die gegebenen Anforderungen abgestimmt sein.

Die Verpackung kann in üblicher Weise mit mindestens einer Aufreißhilfe wie beispielsweise Reißfäden, Sollbruchlinien oder ähnlichem, ausgestattet sein. Bei einer besonders bevorzugten Ausführungsform der Erfindung weist der Verbandstoff mindestens einen Wirkstoff auf. Dabei kann es sich um ein transdermales therapeutisches System, bei dem eine Einzelverpackung unumgänglich ist, handeln.

Nachfolgend wird die Erfindung anhand der begleitenden Zeichnungen näher erläutert. Es zeigt

Fig. 1 den schematischen Querschnitt einer Ausführungsform eines gemäß der Erfindung verpackten Verbandstoffes und

Fig.2 den schematischen Querschnitt einer weiteren Ausführungsform eines erfindungsgemäß verpackten Verbandstoffes.

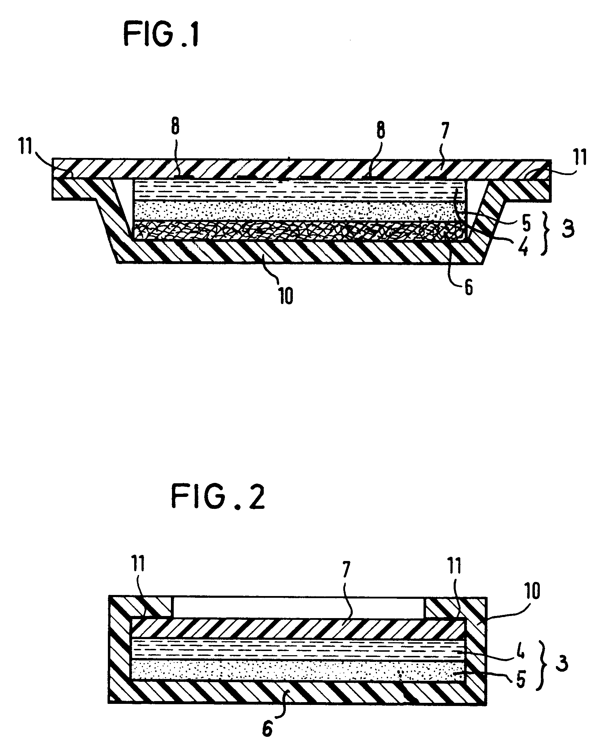

Wie in Fig. 1 gezeigt, ist eine aus textilem Material gebildete Trägerschicht 4 mit einer haftklebenden Schicht 5 beschichtet, die eine pharmakologisch wirksame Substanz enthalten kann und auf der zur Haut gewandten Seite eine Abdeckschicht 6 aufweist, die hier aus einem durch Silikonisierung abhäsiv ausgerüsteten Papier besteht. Die Stützfolie 7 überragt die Trägerschicht 4 an allen Seiten und ist aus einem gegenüber der Trägerschicht starren, mehrschichtigen Folienmaterial ausgebildet. Sie ist durch nach einem vorherbestimmten Muster verteilten Stellen 8 mit der Trägerschicht 4 durch Heißsiegeln verbunden wobei während des Heißsiegelns ein Siegelrand 11 mit der untergelegten Verpackungsfolie 10 ausgebildet wird.

In Figur 2 ist eine weitere bevorzugte Ausführungsform der Erfindung dargestellt bei der die Stützfolie 7 dieselbe Größe besitzt wie die Trägerschicht. Die Verpackungsfolie 10 umschließt den Verbandstoff und ist an den Rändern der Oberseite der Stützfolie 7 mit dieser verklebt. Die Haftklebeschicht 5 besitzt hier die Schutzschicht nicht in gesonderter Form, sondern die Verpackungsfolie 10 enthält die Schutzschicht 6 als integrierter Bestandteil; sie übernimmt also die Funktion der Schutzschicht. Unmittelbar nach Öffnen der Verpackung kann der Verbandstoff appliziert werden.

Nachfolgend wird eine bevorzugte Ausführungsform des erfindungsgemäßen Verbandstoffes anhand eines Beispiels beschrieben:

Beispiel 1:

Herstellung eines verpackten Verbandstoffes wobei ein Teil der Verpackung als Stützfolie fungiert.

Ein Verbandstoff der Größe 15 x 20 cm$^2$, bestehend aus einer textilen Trägerschicht und einer darauf aufgebrachten wirkstoffhaftigen Haftklebeschicht wird mittig zwischen zwei auf jeder Seite etwa 1,5 cm überstehende heißsiegelbare Verpackungsmaterialabschnitte angeordnet. Das Verpackungsmaterial ist so ausgewählt, das der mit der Trägerschicht in Kontakt kommende Abschnitt die Rolle der Stützfolie übernehmen kann. In einem oder zwei aufeinanderfolgenden Schritten wird dann die Randsiegelung und die wieder lösbare Verbindung der Stützfolie mit der Trägerschicht durchgeführt. Die fertigen Siegelrandbeutel werden dann in üblicher Weise konfektioniert.

**Patentansprüche**

1. Primärverpackung für flächenstabilisierte Verbandstoffe, die eine Trägerschicht (4) und eine Haftklebebereiche (5) aufweisende hautseitige Kontaktfläche, die vor Gebrauch des Verbandstoffes durch eine abziehbare Schutzschicht (6) geschützt ist, und eine an der der Haut abgewandten Oberfläche der Trägerschicht (4) ablösbar angebrachte, mindestens einschichtige Stützfolie (7) enthalten, die vom applizierten Verbandstoff entfernbar ist, dadurch gekennzeichnet, daß die Stützfolie (7) oder die Stützfolie (7) und die Schutzschicht (6) jeweils ein Teil einer den Verbandstoff (3) schützenden Verpackung (10) ist.

2. Primärverpackung für flächenstabilisierte Verbandstoffe nach Anspruch 1, dadurch gekennzeichnet, daß die Trägerschicht (4) des Verbandstoffes (3) textiles Material, ein Polymermaterial oder eine Metall enthaltende Schicht ist.

3. Primärverpackung nach einem der Ansprüche 1 oder 2, dadurch gekennzeichnet, daß die Stützfolie (7) ein mindestens einschichtiges Flächengebilde aus Polymer-, Papier- oder Metall enthaltenden Materialien ist.

4. Primärverpackung nach Anspruch 3, dadurch gekennzeichnet, daß die Stützfolie (7) durch zumindest punktweise Heißsiegelung an der Trägerschicht (4) befestigt ist.

5. Primärverpackung nach einem der Ansprüche 1 bis 3, dadurch gekennzeichnet, daß die lösbare Verbindung zwischen Trägerschicht (4) und Stützfolie (7) durch eine mindestens partielle Verklebung, durch andere mechanische oder elektrostatische Kräfte oder durch Schweißvorgänge erzeugt ist.

6. Primärverpackung nach Anspruch 5, dadurch gekennzeichnet, daß die lösbare Verbindung zwischen Trägerschicht (4) und Stützfolie (7) durch Erzeugen der Trägerschicht unmittelbar auf die Stützfolie hergestellt ist.

7. Primärverpackung nach einem der vorangehenden Ansprüche, dadurch gekennzeichnet, daß die Stützfolie (7) flächenmäßig mindestens die Größe der Trägerschicht (4) aufweist.

8. Primärverpackung nach einem oder mehreren der vorangehenden Ansprüche, dadurch gekennzeichnet, daß das den übrigen Teil der Verpackung bildende Flächengebilde im Randbereich an der Stützfolie (7) befestigt ist.

9. Primärverpackung nach einem der vorangehenden Ansprüche, dadurch gekennzeichnet, daß der verpackte Verbandstoff mindestens einen Wirkstoff aufweist.

10. Primärverpackung nach einem der vorangehenden Ansprüche, dadurch gekennzeichnet, daß der verpackte Verbandstoff ein transdermales therapeutisches System ist.

## Claims

1. Primary packaging for surface-stabilised dressing materials, which include a base layer (4) and a skin-side contact surface, having contact adhesive regions (5), which contact surface is protected before use of the dressing material by a peelable protective layer (6), and includes an at least single-layer supporting film (7), which is releasably attached to the surface of the base layer (4) facing away from the skin and can be removed from the applied dressing material, characterised in that the supporting film (7) or the supporting film (7) and the protective layer (6) is respectively part of a packaging (10) protecting the dressing material (3).

2. Primary packaging for surface-stabilised dressing materials according to Claim 1, characterised in that the base layer (4) of the dressing material (3) is textile material, a polymer material or a layer containing metal.

3. Primary packaging according to one of Claims 1 or 2, characterised in that the supporting film (7) is an at least single-layer sheet-like structure of materials containing polymer, paper or metal.

4. Primary packaging according to Claim 3, characterised in that the supporting film (7) is fastened by at least punctiform heat sealing to the base layer (4).

5. Primary packaging according to one of Claims 1 to 3, characterised in that the releasable connection between base layer (4) and supporting film (7) is produced by an at least partial adhesive bonding, by other mechanical or electrostatic forces or by welding operations.

6. Primary packaging according to Claim 5, characterised in that the releasable connection between base layer (4) and supporting film (7) is established by producing the base layer directly on the supporting film.

7. Primary packaging according to one of the preceding claims, characterised in that the supporting film (7) has a surface area at least the size of the base layer (4).

8. Primary packaging according to one or more of the preceding claims, characterised in that the sheet-like structure forming the remaining part of the packaging is fastened in the marginal area to the supporting film (7).

9. Primary packaging according to one of the preceding claims, characterised in that the packed dressing material has at least one active agent.

10. Primary packaging according to one of the preceding claims, characterised in that the packed dressing material is a transdermal therapeutic system.

## Revendications

1. Emballage primaire pour bandages à surface stabilisée, qui comportent une couche de support (4) et une surface de contact côté peau présentant des zones autocollantes (5), laquelle surface est protégée avant usage du bandage par une couche de protection enlevable (6), et une feuille d'appui (7) au moins monocouche qui est agencée de manière détachable sur la surface de la couche de support (1) opposée à la peau, laquelle couche d'appui peut être séparée du bandage appliqué, l'emballage étant caractérisé en ce que la feuille d'appui (7) ou la feuille d'appui (7) et la feuille de protection (6), respectivement, constitue(ent) une partie d'un emballage (10) protégant le bandage (3).

2. Emballage primaire pour bandages à surface stabilisée selon la revendication 1, caractérisé en ce que la couche de support (4) du bandage (3) est constituée d'une couche de matière textile, d'une matière polymère ou d'une couche contenant du métal.

3. Emballage primaire selon l'une quelconque des revendications 1 et 2, caractérisé en ce que la feuille d'appui (7) est constituée d'un produit plat au moins monocouche formé de matières polymères, de papier ou de matières contenant du métal.

4. Emballage primaire selon la revendication 3, caractérisé en ce que la feuille d'appui (7) est fixée à la couche de support (4) au moins ponctuellement par scellage à chaud.

5. Emballage primaire selon l'une quelconque des revendications 1 à 3, caractérisé en ce que la liaison détachable entre la couche de support (4) et la couche d'appui (7) est produite par un collage au moins partiel, par d'autres forces mécaniques ou électrostatiques ou par soudage.

6. Emballage primaire selon la revendication 5, caractérisé en ce que la liaison détachable entre la couche de support (4) et la feuille d'appui (7) est formée par production de la couche de support (4) directement sur la feuille d'appui (7).

7. Emballage primaire selon l'une quelconque des revendications précédentes, caractérisé en ce que la feuille d'appui (7) présente au moins la même surface que la couche de support (4).

8. Emballage primaire selon l'une et/ou l'autre des revendications précédentes, caractérisé en ce que le produit plat formant la partie restante de l'emballage est fixée à la feuille d'appui (7) dans la zone marginale.

9. Emballage primaire selon l'une quelconque des revendications précédentes, caractérisé en ce que le bandage emballé présente au moins un principe actif.

10. Emballage primaire selon l'une quelconque des revendications précédentes, caractérisé en ce que le bandage emballé est un système thérapeutique transdermique.

# FIG .1

# FIG .2